# EUROPEAN PATENT SPECIFICATION

(11) **EP 3 263 147 B1**
(45) Date of publication and mention of the grant of the patent: **28.07.2021**
(21) Application number: 17178827.6
(22) Date of filing: 29.06.2017
(51) Int. Cl.: A61L 31/08, A61B 5/00, A61L 31/14

(54) **IMPLANTABLE DEVICE HAVING AN OUTER SURFACE COMPRISING GOLD AND ITS USE AS AN ANTIMIGRATION DEVICE**
IMPLANTIERBARE VORRICHTUNG MIT AUSSENOBERFLÄCHE MIT GOLD UND DEREN VERWENDUNG ALS ANTIMIGRATIONSVORRICHTUNG
DISPOSITIF IMPLANTABLE PRÉSENTANT UNE SURFACE EXTÉRIEURE COMPRENANT DE L'OR ET SON UTILISATION COMME DISPOSITIF ANTI-MIGRATION

(30) Priority: 29.06.2016 EP 16176882
(43) Date of publication of application: 03.01.2018
(73) Proprietor: Safe Implant Technology ApS, 2920 Charlottenlund (DK)
(72) Inventor: DANSCHER, Gorm, 8000 Aarhus C (DK)
(74) Representative: AWA Denmark A/S

(56) References cited:
- US-A1- 2004 151 938
- US-A1- 2012 238 842
- US-A1- 2013 267 962

## Description

### Introduction

The present invention relates to an implantable device, in particular a microchip comprising a core portion and a capsule encapsulating the core portion wherein at least a portion of the outer surface comprises gold having a purity greater than 99.00% w/w and is for use in a method of reducing local immune response and thereby stop the migration of the implant.

### Background art

A microchip, sometimes also referred to as a transponder or simply a marker, is a small electronic device which can be implanted in humans and animals. Microchips are presently used for multiple purposes, such as tracking and identification, and have even more potential uses, such as data storage, data sharing, and security.

The use of microchips as identification markers in pets and domestic animals is well known and widely used, whereas the use of microchips in humans is still very limited.

In general, it is a requirement that implantable devices and electronic microchips are encapsulated to isolate the electronic components from body fluids present in the receiving subject. The encapsulation material must be biocompatible and completely non-adverse to the surrounding tissue at the implant site.

Generally, implanted microchips do not cause severe damage to the host, however different adverse reactions and complications have been documented. For instance, it is known that microchips can migrate to a different location in the receiving object during its lifetime. Dislocated microchips may pose a serious threat to the subject, as they may migrate into positions where they hinder the mobility of the subject. Also, if a migrated microchip ends up in a location where it is subjected to undue mechanical stress or repeated impacts it may break causing even more damage. In some instances microchips have even migrated out of the receiving subject, which has its obvious drawbacks.

Multiple attempts have been made in order to prevent migration of implanted microchips. US 5,074,318 describes a marker adapted to be injected subcutaneously into a laboratory animal, this marker is provided with a partial coating having a high coefficient of friction, for example Silastic® which is a silicone elastomer, and polypropylene are mentioned as coating materials, as they both have high coefficients of friction. The coating thus provides a high friction to the marker thereby preventing it from migrating. It is also suggested that markers with glass surfaces can be etched to make the surface more uneven and thereby increasing the friction of the surface, however this approach was found to weaken the glass.

In US 5,840,148 a different approach for providing anti-migration means is described by which the microchip is provided with an anti-migration cap. The anti-migration cap is described as having projections, or being coated as described in US 5,074,318.

EP 1 228 686 describes an implantable marker with anti-migration means in the form of a fixation structure. The fixation structure, described in EP 1 228 676 provides a significantly increased total surface of the marker. An example is given, wherein a number of filaments are provided on the surface of the microchip, which improve the integration of the microchip into the surrounding tissue.

US2013/267962 describes an implantable chip surrounded by a protective capsule, wherein barbs on the outer surface provide means for against migration. The capsule prevents damage to the device from inflammatory reactions.

US 2012/0238842 describes an implantable device, preferably a sensor for measuring glucose levels in the body having a coating protecting said implantable device against inflammation reactions. The coating may be silver, platinum, palladium, manganese, gold, and alloys and/or oxides thereof.

US2004/151938 discloses a method for implantation of heavy metals such as gold.

The prior art in the field of preventing microchip migration, is generally focused on altering the surface structure, in order to increase the mechanical features of the microchip. But altering the surface to provide mechanical friction, may be cumbersome and may even decrease the strength of the surface material (as has been described in US 5,074,318 regarding etching of surface glass). Adding additional mechanical features, such as sharp projections or increasing the surface area of the microchip with filaments, may provide an unnecessary large microchip, which is more complicated to insert in the receiving subject, and may cause more irritation once implanted.

On this background it is an object of the present invention to provide a device, such as a microchip, which overcomes one or more of the drawbacks of the prior art.

### Summary of the invention

This and further objects are achieved by in a first aspect providing an implantable device comprising a core portion and a capsule encapsulating the core portion, said capsule having an outer surface, wherein at least a portion of the outer surface comprises gold having a purity greater than 99.00% w/w, preferably 99.99% w/w and being for use in a method of reducing local immune response and thereby stop the migration of the implant. The implantable device may be an implantable microchip comprising a core portion with electrical components, and a capsule encapsulating the core portion, said capsule having an outer surface, wherein at least a portion of the outer surface is gold.

In the context of the present invention gold is pure gold which in this context is defined as a purity greater than 99.00% w/w, preferably 99.9% w/w gold.

A device having a surface of pure gold according to the present invention provides local reduction of an immune response, due to the dissolucytosis of gold at the outer surface portion of the device. The device according to the present invention is able to limit the local inflammation around the implanted device or microchip i.e. the foreign body reaction, thereby preventing migration of the device.

It is believed that fluctuating local inflammation along the surface of foreign bodies is the cause of foreign body migration and it is known that metallic gold suppresses inflammation.

If gold constitutes the whole surface or part of it, or is amalgamated into the surface of the foreign body, gold ions released by dissolucytosis will suppress or prevent the points of inflammation along the surface of the foreign body and thereby securing that the foreign body (such as a microchip) does not migrate out of position. The pure gold may be gilded or spotted onto the outer surface of the implantable device, or, the surface may be in the form of an outer net or mesh of pure gold encapsulating the device.

Gold suitable for use in the present invention is pure gold having a purity greater than 99.00% w/w, most preferred 99.9% w/w or greater. Impurities can cause toxic effects on the tissue and therefore the gold according to the invention is of high purity.

In a preferred embodiment the device is an encapsulated microchip and the core portion comprises electrical components. In this embodiment the capsule or housing encapsulating the electrical components may be made of glass, polymer material such as epoxy composite or polypropylene polymer, ceramics or other biocompatible material. Sometimes a combination of the different materials is used. The capsule functions to protect the electronic components from body fluid penetration and tissue invasion to the system and is thus hermetically sealed upon assembly of the components. Hence, in an embodiment of the invention the capsule is a solid shell.

In the context of the present invention solid shell should be understood as the capsule being impermeable to liquid and body fluid, such as water and blood.

Having a solution where the migration of microchips can be effectively prevented is of high commercial interest since it would increase the application possibilities and receiver compliance of microchips dramatically.

Upon implantation of a device or microchip, without the wish to be bound by any theory it is believed that local macrophages will attach themselves to the surface of the microchip and produce an ultra-thin layer, a dissolution membrane, within which the macrophages may control the chemical milieu. If metallic gold constitutes the whole or part of the surface, dissolucytosis of gold ions will take place. The dissolucytosis of gold ions within the dissolution membrane is most likely caused by the capability of the macrophages to release cyanide ions and alter the oxygen tension and the pH in their vicinity *(*Larsen A, Stoltenberg M. & Danscher, G (2007) In vitro liberation of charged gold atoms. Autometallographic tracing of gold ions released by macrophages grown on metallic gold surfaces 128, 1-6 Histochem Cell Biol.*;* Ferre N, Claria, J (2006) New insight into the regulation of liver inflammation and oxidative stress. Mini Rev. Med. Chem. 6, 1321-1330*.).* The macrophages release cyanide into the dissolution membrane, and the following chemical process occurs:

4Au + 8CN⁻ + 2H₂O + O₂ = 4[Au(CN)₂]⁻ + 4OH⁻

The complex ion aurocyanide Au(CN)₂⁻, which is a relatively stable ion, known to inhibit several of the molecular components of inflammation, decreases or halt the number of inflammatory cells *in situ.* It is believed that this reduction of the local immune response causes the stop of migration of gold implants.

A further advantage of using pure gold according to the invention is that gold ions liberated from the macrophages do not spread further away than about 500 microns from the locality of where the gold is present on a portion of the outer surface of the capsule. Hence, the risk of systemic exposure is negligible i.e. non existent.

In an embodiment of the present invention, at least a part of the outer surface portion is provided as a coating layer. The coating layer according to this embodiment need not to be coherent, and may be provided in any suitable pattern. A non-coherent or non-continuous pattern may be advantageous since less gold coating material is needed. By providing at least a part of the outer surface portion as a coating, the outer surface portion can easily be applied to a commercially available microchip by coating a microchip or device with gold. Hence, the cost of producing the implant according to the invention may be reduced.

In a preferred embodiment the pure gold is provided as a coating covering substantially the whole outer surface of the capsule.

In the context of the present application substantially the whole outer surface should be understood as the coating covers the outer surface of the capsule in such a way that during insertion of the device, the shape and placement of said device is irrelevant for the ability of the device to prevent migration as long as the outer surface of the capsule faces the surrounding tissue.

As an example if the pure gold coating is provided as dots, each neighbouring dot will be provided with a distance to each other, which is low enough to ensure that the device cannot migrate independent on the shape and placement of the device in the body. The exact distance between dots is within the skill of the art and depends on the shape, size, and properties of the device.

Hence, in a further embodiment, at least a part of the coated outer surface portion is provided as dots of pure gold of predetermined sizes. Applying the noble metal, such as pure gold, onto the microchip in the form of dots is advantageous due to the ease of application. Where dots are applied onto the surface of the device, this is to be understood as a non-coherent or non-continuous coating. Coating in the form of gold dots also has advantages similar to other non-coherent or non-continuous patterns, since less gold is necessary. Dots of predetermined sizes is also highly customizable, the number, position, and individual size of dots may be varied from case to case, according to the specific needs.

In a further embodiment the coating is in the form of patchy layers i.e multiple non-continuous layers that is provided on a portion of the outer surface of the capsule.

As each layer is not provided continually it may be possible to limit the amount of pure gold needed to fixate the implantable device according to the invention. Furthermore, the thickness and/or surface roughness of each patchy layer may be independently selected so as to improve mechanical fixation of the implantable device.

In an embodiment of the invention, the coating is in the form of patchy layers and dots.

In an embodiment of the invention the amount of pure gold is selected on the basis of the total surface area of the capsule.

In a preferred embodiment the amount of pure gold deposited on the surface of the surface area of the capsule is up to 50 nm thick.

In a further embodiment, the part of the outer surface portion of pure gold, which in a preferred embodiment is a coating, has a thickness from 10 nm to 4 µm, such as from 50 nm to 3 µm, from 60 nm to 20 µm, from 70 nm to 20 µm, from 100 nm to 20 µm, from 200 nm to 800 nm, or from 300 nm to 500 nm. The thickness of the coating may be the same over the entire coating, or in a particular embodiment the coating may have a varying thickness over the outer surface portion. A varying thickness of the outer surface portion may be advantageous where different surface structures are needed, such as a fluted or grooved surface to mechanically improve immobility the foreign body in the tissue.

It has been found that the combination of pure gold distributed on the surface area of the capsule in the abovementioned range provides a durable fixation of the implantable device while still being cost-efficient.

The implantable device or microchip according to anyone of the preceding embodiments and further aspects of the invention may be produced by a method, comprising the steps of: a) providing an implantable device comprising a core portion with electrical components, and a capsule encapsulating the core portion, said capsule having an outer surface, and b) applying a noble metal, preferably gold, onto the outer surface of the microchip provided in step a), thereby providing an outer surface portion capable of preventing migration of the microchip when implanted in a subject. Preferably the application is achieved by coating.

In an embodiment, at least a part of the outer surface portion is provided as a pure gold coating, said pure gold coating being provided by spraying or otherwise applying pure gold onto the surface of the microchip. Multiple different application processes may be used, including but not limited to various gold plating techniques, gilding techniques, and deposition techniques, e.g. physical vapor deposition, mechanical gilding, chemical gilding, cold gilding, wet gilding, fire-gilding and depletion gilding, amongst other techniques of applying gold to an object, which would be known to the person skilled in such art. The device may thus be any device onto which gold is applied.

It is contemplated that the use of pure gold as a coating or surface constituent according to the present invention may also be applicable on other implants or foreign bodies such as stents, hearing aids and pacemakers which are to be implanted in a subject, to provide anti-inflammatory and anti-migration qualities.

Disclosed herein is an implantable device comprising a core portion for example made of electrical components, and a capsule encapsulating the core portion, said capsule having an outer surface, wherein at least a portion of the outer surface is of a noble metal, for use as an anti-migrating microchip in the prevention of adverse effects associated with migrating implanted foreign bodies.

Yet another aspect of the invention relates to a method for the prevention of migration of an implantable device, the method comprising the steps of:
a) providing an implantable device comprising a core portion, and a capsule encapsulating the core portion, said capsule having an outer surface, b) applying pure gold on the outer surface of the device provided in step a), thereby providing an outer surface portion preventing migration of the device when implanted in a subject, and c) implanting the microchip into said subject at a predetermined location in the subject.

In an embodiment of the above disclosed aspect, the subject is a human or a wild or a domestic animal or a pet.

Yet another aspect of the invention relates to the use of an implantable microchip comprising a core portion with electrical components, and a capsule encapsulating the core portion, said capsule having an outer surface, wherein at least a portion of the outer surface is of pure gold, for the prevention of adverse effects associated with migration of an implanted microchip in a subject including suppression of inflammation caused release of toxic metals from the capsule.

Further objects and advantages of the invention will be obvious or apparent from the exemplified embodiments, cases, and studies described in detail hereinafter.

### Brief description of the drawings

The drawings described in the following are to support the detailed description. The invention will be described with reference to the drawings in which:
Figs. 1 A-C show x-ray photographs of a dog with implanted pure gold, around the knee. Fig A is immediately after implantation, whereas Fig B was taken about one year after implantation.
Figs. 2 shows x-ray photographs of a 45-year old man having implanted pure gold in the lower lumbar region at the age of 12.
Figs. 3 A and B show X-ray photographs of a patient with an implant immediately after implantation: A and after 8 months: B.

### Detailed description of the invention

The device according to the present invention may be any device for implantation into an animal, such as a mammal. In particular the device may be a microchip, microimplants, gold threads and needles used e.g. for stability after injuries.

In a particular embodiment the device is a microchip which may be produced from any commercially available microchip/transponder which is coatable, e.g. "ID-100A Microtransponder" from trovan®.

The microchip according to an embodiment of the present invention can be implanted by use of a properly sized syringe with a plunger (e.g. the "IM-200 Syringe Implanter" from trovan®), or by any commercially available implanter of a size suitable for the respective microchip.

Migration of a device according to the invention is to be understood as a device which has moved away from its initial or intended implant site. The specific distance regarded as migration may vary depending on the position in the body into which the microchip is implanted. However, it is contemplated that any movement of a microchip, which is further than 5 cm from implant position, should be regarded as an inadvertent migration in the present context.

In the context of the present invention dissolucytosis is intended to mean liberation of gold ions, from gold of the outer surface portion.

The invention will now be explained in greater detail with reference to a microchip embodying the device. The invention should however not be limited to a microchip and devices according to the invention may be prepared in a similar manner.

An implantable microchip according to the invention may be prepared by coating a commercially available implantable microchip with a 10 - 80 nm thin layer of pure gold using the nonreactive physical vapor deposition (PVD) process named magnetron sputtering. Magnetron sputtering is a magnet field superimposed on a sputtering configuration. The superimposed magnetic field induces an additional force on the charged particles. The force is perpendicular to the speed direction, whereby the charged particles are forced to circulate in a spiral instead of moving in straight lines to the gold target, where the gold for the coating process is placed. The longer pathway increases the number of collisions between mainly electrons and ions and enables the plasma to be self-sustaining at a lower working pressure. The higher collision energy between the target and charged gas ions will apply a higher energy to the sputtered gold atoms and improve the adhesion to the porous surface on the implants.

Another contemplated preparation of a device according to the invention, is as follows: Commercially available implantable devices, such as a microchip, may be electrolytically cleaned by cathodic polarization of the device for a heavy formation of hydrogen gas at the surface. The treatment is carried out in a strong alkaline solution containing e.g. NaOH. Each microchip is then immersed in a weak acid solution (e.g. 5% H₂SO₄), neutralizing the alkaline film at the surface. Initial electrolytic strike plating with gold (∼0.5 µm) is carried out in an acid gold electrolyte based upon a gold tetrachloride complex AuCl₄ - to improve the adhesion of the following gold layer. After assuring that the initial strike plating is made properly upon the surface, a pure gold coating with low mechanical stress is applied by deposition of a 3- to 4-µm-thick gold layer from a weak acid gold bath based upon AuCN. Between each of the process steps, careful rinse in pure water is carried out to avoid contamination.

It is contemplated that different thicknesses and patterns may be optimal in different cases, e.g. based on the type and size of subject, the implant position in the subject, etc. The anti-migration effects of gold ions are dependent on the amount of gold surface available for attack by macrophages. Thus, it is an advantage to provide a gold device with a surface as large as possible and further when the surface is large the layer of gold may be provided as thin as possible from an economical perspective.

The outer surface portion may be produced as a capsule or housing for the device thus being detachable from the inner core of the device such as a cover, lid or one or more separate pieces of noble metal which is kept on or around the microchip. In an embodiment it is contemplated that a thin gold wire is spun around the device, thus being a separate piece not directly attached to the device but rather associated with the device, and thus still prevented from leaving the microchip due to its geometry.

In a further embodiment of the invention a capsule made from a polymeric material completely encapsulating a microchip so as body liquids cannot come into contact with the electrical components of the microchip. The capsule is covered by a coating layer of high purity gold. The coating is provided as dots having a thickness between 10 nm to 4 µm and the distance between each dot is small enough (approx. 100 my) to prevent the device from migrating independent on the direction of the device in the body.

In an embodiment similar to the above described, the coating may preferably be provided as a continuous coating layer covering the whole outer surface of the capsule and having a thickness of between 10 nm to 4.

In an embodiment of the invention, the coating of pure gold is applied and/or adhered to the surface of the device by dipping or submerging the implant into a physiological acceptable solution e.g. comprising hyaluronic acid and at least 0.1 g/L of gold particles according to the invention, which corresponds to approximately 7,200,000 gold particles. Preferably the concentration of gold particles is 5 g/L gold. The gold particles are of a purity above 99.00% w/w, preferably 99.99% w/w and are sized from 20 to 100 microns. The 0.1 g/L concentration of gold particles results in approximately 36 gold particles per cubic millimetre of coating solution. After dipping in the coating solution comprising gold particles according to the invention, the gold coated-implant is thus retrieved from the solution and allowed to dry. The implant may be placed in sterile wrapping until use or directly used implanted into the human body.

The invention will now be illustrated by way of the following examples describing the use of gold implants.

### Example 1

A study supporting the invention has been conducted with 10 dogs aged between 5.5 months and 7 years and 6 months, and weighing between 8 and 54 kg, attending the veterinary hospital because of limping walk were diagnosed with osteoarthritis in one or more joints. Two of the dogs were females, 5 males and 3 neutered. Prior to diagnosis a detailed anamnesis was taken from the owner and the animals were inspected and palpated before X-ray photographs were taken of the involved joints.

The animals were fully anaesthetized after premedication with Atropin®, Plegicil® and metadon®. Propofol® was injected followed by intubation and inhalation anesthetization with Isofluran®.

Gold implants, tiny oval balls consisting of a 300 mm long and 30 micron thick thread of 99.99% pure gold, were placed adjacent to the diseased joint i.e. in the capsule and surrounding looser connective tissue, but never in the joint cavity.

The area around the joint to be treated was prepared as for surgery and the needles placed as close as possible to the joint. For treatment of hip joints facet joints of big dogs (20-25 kg body weight) needles of 14 G with a length of 60 mm were used. In all cases X-ray photographs were taken when the needles have been placed, in order to ensure correct positioning before the gold implants were placed in the needle and pushed through the needle with a stiletto.

For treatment of smaller dogs and joints close to the surface of bigger dogs, 14G with a length of 38 mm were used.

In contrast to hip and spine implantations, X-ray photographs were not taken when injecting gold implants in elbow and knee joints, where a direct palpation of the needles could take place.

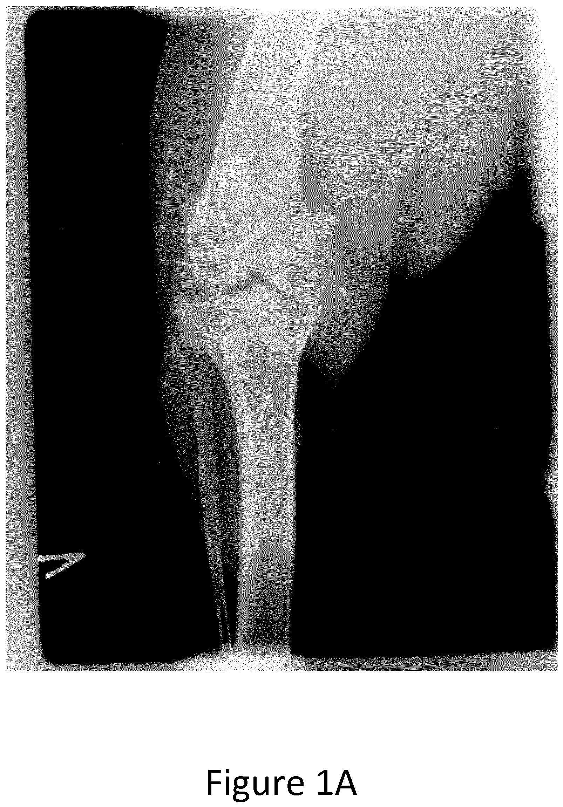

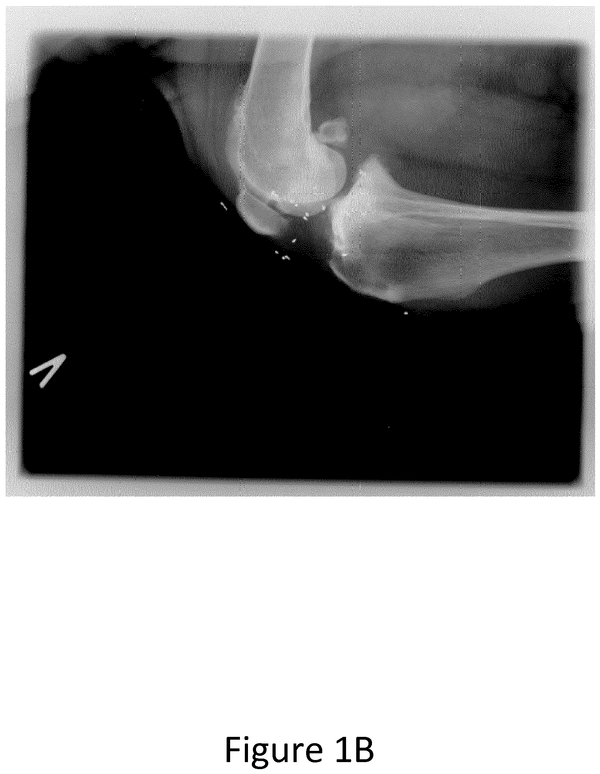

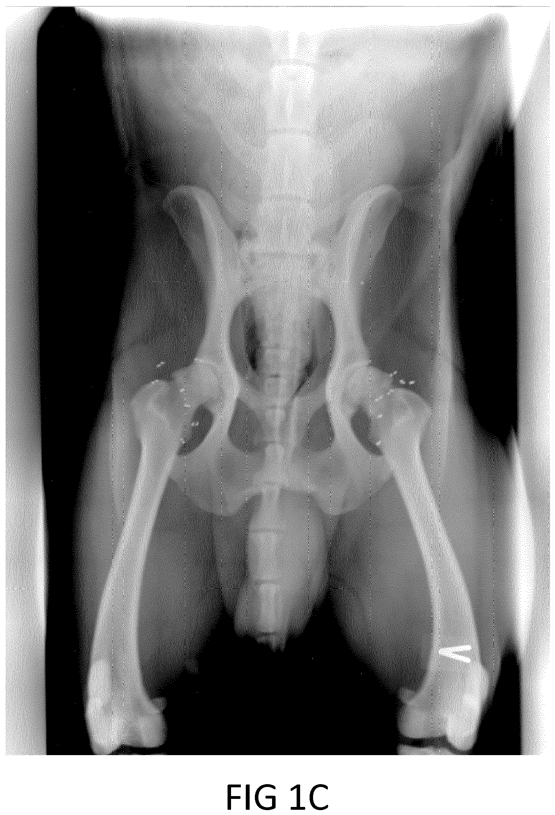

Immediately after the 'auromedication' X-ray photographs were taken of the implant-receiving joints, in order to determine the exact location of the gold implants. In a time span varying from 12 to 35 months after the 'auromedication', the dogs were X-ray-photographed again using the exact same projections as used at the first X-ray, figs. 1A-C show such photographs of the same dog.

By comparing the X-ray photographs, any migration of the gold implants would be visible as a difference between the photographs.

Referring again to figures 1A-C showing the X-ray photographs of the dog implanted with a number of gold beads after approximately 1 year, each picture visualizes the position of the implanted gold beads from a different angle. The implanted gold beads are visible as small white dots on each of the pictures. From the pictures it is apparent that the gold beads are still located around the hip joints where they were implanted about a year earlier.

The results of this study, although the implants were tiny oval balls consisting of a 300 mm long and 30 micron thick thread of 99,99% pure gold, is thought to be representative of any gold coated object, such as a gilded or gold coated microchip, since the activity is provided by the pure gold accessible on the surface.

### Example 2

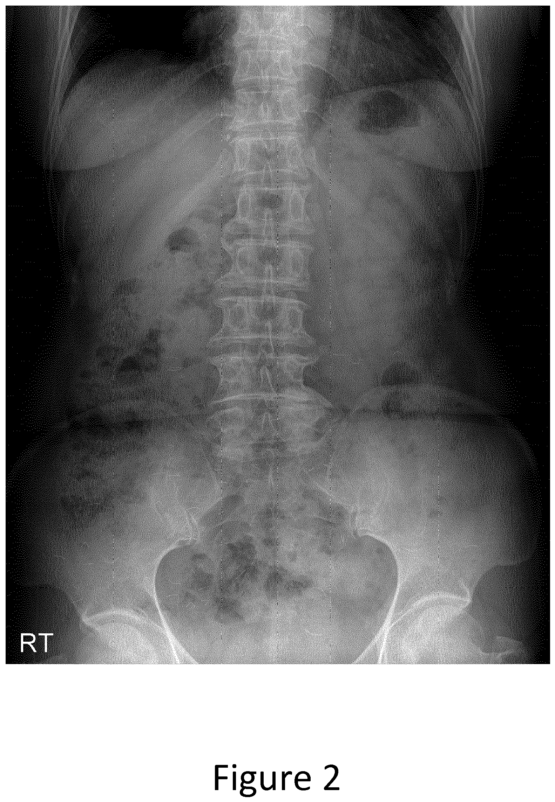

In a study on humans, patients that have been subjected to gold implantation were analyzed for migration of the implants. The study supported the findings in the abovementioned study on dogs. In one example a 45 year old man had small gold threads implanted in his back at the age of 12 years. At the age of 12 year he had a serious back injury in the lumbar region and got acupuncture where small threads were placed as seen in the X-ray radiographs in figure 2. The radiographs of the 45 year old man show that the gold treads are still located in the region where they were injected (figure 2).

### Example 3

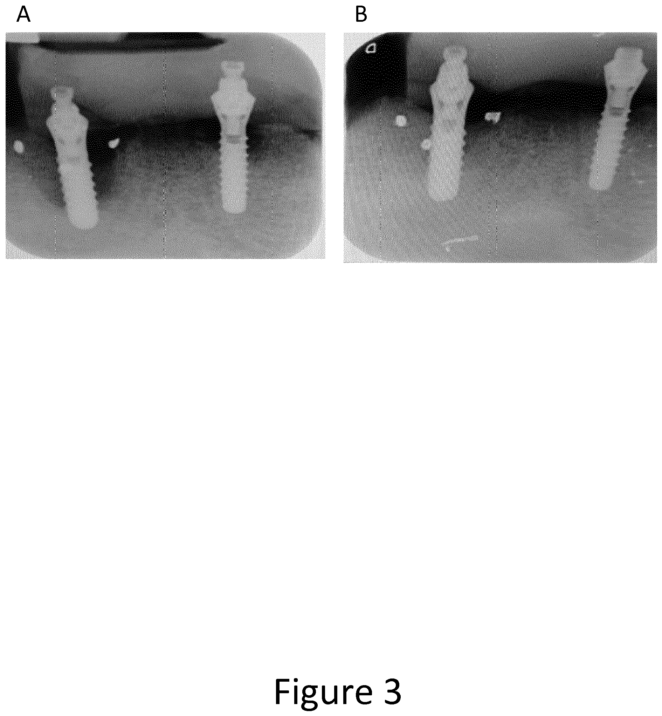

In another example according to the invention a patient having a dental implant was treated for paradentosis by the insertion of gold implants, made up of a ball of a 50 micron thick wire of 99.99 % pure gold, having a cross-section of 1000 µm. The gold implants/particles were injected into the periodontium in proximity to the gold implant as shown in figure 3A. Figure 3B shows the position of the gold particles after 8 months. Neither the gold implants nor the dental implants had moved position in the period of time. A visible regrowth of bone had taken place. Thus, it has been shown that gold implants according to the invention strongly fixates to the surrounding tissue.

## Claims

1. An implantable device comprising a core portion and a capsule encapsulating the core portion, said capsule having an outer surface, wherein at least a portion of the outer surface comprises gold having a purity greater than 99.00% w/w, preferably 99.99% w/w and being for use in a method of reducing local immune response and thereby stop the migration of the implant.

2. An implantable device according to claim 1 for use in a method of reducing local immune response and thereby stop the migration of the implant, wherein the gold is provided as a coating substantially covering the whole outer surface of the capsule.

3. An implantable device according claims 1 to 2 for use in a method of reducing local immune response and thereby stop the migration of the implant, wherein the coating is provided as a patchy layer, continuous layer, wire and/or dots of predetermined sizes of pure gold.

4. An implantable device according to claims 1 to 3 for use in a method of reducing local immune response and thereby stop the migration of the implant, wherein the capsule is a solid shell.

5. An implantable device according to claim 4 for use in a method of reducing local immune response and thereby stop the migration of the implant, wherein the implantable device is an encapsulated microchip and the core portion comprises electrical components.

6. An implantable device according to anyone of the preceding claims 1 to 5 for use in a method of reducing local immune response and thereby stop the migration of the implant, wherein the thickness of the coating can be uniformly or heterogeneously distributed across the outer surface of the capsule in the range from 10 0 nm to 4 µm, preferably from 50 nm to 3 µm, from 60 nm to 2 µm, from 70 nm to 1 µm, from 100 nm to 1 µm, from 200 nm to 800 nm, or from 300 nm to 500 nm.

## Patentansprüche

1. Implantierbare Vorrichtung, die einen Kernteil und eine den Kernteil einkapselnde Kapsel umfasst, wobei die Kapsel eine äußere Oberfläche aufweist, wobei mindestens ein Teil der äußeren Oberfläche Gold mit einer Reinheit von mehr als 99,00 Gew.-%, vorzugsweise 99,99 Gew.-% umfasst, und die für die Verwendung bei einem Verfahren, bei dem eine lokale Immunantwort verringert und dadurch das Abwandern des Implantats verhindert wird, bestimmt ist.

2. Implantierbare Vorrichtung nach Anspruch 1 zur Verwendung bei einem Verfahren, bei dem eine lokale Immunantwort verringert und dadurch das Abwandern des Implantats verhindert wird, wobei das Gold als eine Beschichtung bereitgestellt wird, die die gesamte äußere Oberfläche der Kapsel weitgehend bedeckt.

3. Implantierbare Vorrichtung nach Anspruch 1 bis 2 zur Verwendung bei einem Verfahren, bei dem eine lokale Immunantwort verringert und dadurch das Abwandern des Implantats verhindert wird, wobei die Beschichtung als lückenhafte Schicht, durchgehende Schicht, Draht und/oder Punkte mit vorgegebener Größe von reinem Gold bereitgestellt wird.

4. Implantierbare Vorrichtung nach Anspruch 1 bis 3 zur Verwendung bei einem Verfahren, bei dem eine lokale Immunantwort verringert und dadurch das Abwandern des Implantats verhindert wird, wobei es sich bei der Kapsel um eine feste Hülle handelt.

5. Implantierbare Vorrichtung nach Anspruch 4 zur Verwendung bei einem Verfahren, bei dem eine lokale Immunantwort verringert und dadurch das Abwandern des Implantats verhindert wird, wobei es sich bei der implantierbaren Vorrichtung um einen verkapselten Mikrochip handelt und der Kernteil elektrische Komponenten umfasst.

6. Implantierbare Vorrichtung nach einem der vorhergehenden Ansprüche 1 bis 5 zur Verwendung bei einem Verfahren, bei dem eine lokale Immunantwort verringert und dadurch das Abwandern des Implantats verhindert wird, wobei die Dicke der Beschichtung gleichmäßig oder ungleichmäßig im Bereich von 10 0 nm bis 4 µm, vorzugsweise von 50 nm bis 3 µm, von 60 nm bis 2 µm, von 70 nm bis 1 µm, von 100 nm bis 1 µm, von 200 nm bis 800 nm oder von 300 nm bis 500 nm über die äußere Oberfläche der Kapsel verteilt sein kann.

## Revendications

1. Dispositif implantable comprenant une partie de cœur et une capsule encapsulant la partie de cœur, ladite capsule possédant une surface externe, au moins une partie de la surface externe comprenant de l'or possédant une pureté supérieure à 99,00 % p/p, préférablement 99,99 % p/p et étant destiné à une utilisation dans un procédé de réduction de la réponse immunitaire locale et ainsi stopper la migration de l'implant.

2. Dispositif implantable selon la revendication 1 destiné à une utilisation dans un procédé de réduction de la réponse immunitaire locale et ainsi stopper la migration de l'implant, l'or étant mis à disposition en tant que revêtement recouvrant sensiblement la totalité de la surface externe de la capsule.

3. Dispositif implantable selon les revendications 1 et 2 destiné à une utilisation dans un procédé de réduction de la réponse immunitaire locale et ainsi stopper la migration de l'implant, le revêtement étant mis à disposition comme une couche inégale, une couche continue, un fil et/ou des points de tailles prédéterminées d'or pur.

4. Dispositif implantable selon les revendications 1 à 3 destiné à une utilisation dans un procédé de réduction de la réponse immunitaire locale et ainsi stopper la migration de l'implant, la capsule étant une enveloppe solide.

5. Dispositif implantable selon la revendication 4 destiné à une utilisation dans un procédé de réduction de la réponse immunitaire locale et ainsi stopper la migration de l'implant, le dispositif implantable étant une micropuce encapsulée et la partie de cœur comprenant des composants électriques.

6. Dispositif implantable selon l'une quelconque des revendications précédentes 1 à 5 destiné à une utilisation dans un procédé de réduction de la réponse immunitaire locale et ainsi stopper la migration de l'implant, l'épaisseur du revêtement pouvant être distribuée de manière uniforme ou hétérogène sur la surface externe de la capsule dans la plage de 10 0 nm à 4 µm, préférablement de 50 nm à 3 µm, de 60 nm à 2 µm, de 70 nm à 1 µm, de 100 nm à 1 µm, de 200 nm à 800 nm, ou de 300 nm à 500 nm.
